# FASCICULE DE BREVET EUROPEEN

(11) **EP 1 935 934 B1**
(45) Date de publication et mention de la délivrance du brevet: **18.07.2012**
(21) Numéro de dépôt: 06292039.2
(22) Date de dépôt: 21.12.2006
(51) Int. Cl.: C08K 5/20, C09K 3/10

(54) **Additif de rhéologie sous forme de pâte pré-activée**
Rheologieadditiv in Form voraktivierter Paste
Rheology additive in the form of a pre-activated paste

(43) Date de publication de la demande: 25.06.2008
(73) Titulaire: Arkema France, 92700 Colombes (FR)
(72) Inventeur: Caron, Sébastien, 60134 Montreuil sur Therain (FR); Pires, Elizabeth, 60100 Creil (FR); Rimmer, Susan, 60500 Chantilly (FR); Roussel, Joël, 60610 Lacroix Saint Ouen (FR); Trang, Yohann, 69004 Lyon (FR)
(74) Mandataire: Killis, Andréas

(56) Documents cités:
- JP-A- 63 015 876
- US-A- 4 462 926
- US-A- 5 373 042

## Description

La présente invention concerne des additifs de rhéologie sous forme de pâte pré-activée, un procédé spécifique de préparation de ces additifs, des compositions spécifiques comprenant ledit additif, ainsi qu'un procédé spécifique de préparation desdites compositions, des utilisations spécifiques et des revêtements spécifiques obtenus à partir desdits additifs. Ces additifs spécifiques permettent de modifier la viscosité de compositions de mastics, de colles, d'adhésifs, de revêtements, tels que les peintures, vernis, gel coats, encres, ou de compositions de moulage.

Il est connu de l'état de l'art des compositions d'agents d'étanchéité, d'adhésifs ou de revêtements à base de polyuréthane comprenant un additif de type polyamide non réactif, pour ajuster la viscosité desdites compositions. En effet, EP 467 533 décrit un polyamide obtenu à partir d'au moins un acide polycarboxyhque choisi parmi l'acide sébacique, l'acide azélaïque, l'acide dodécane dioïque et les dimères ou trimères d'acides gras, avec au moins un composé comprenant un groupe -NH₂ ou -NH choisi parmi les monoamines primaires et secondaires, naturelles et synthétiques, saturées ou insaturées, soit à insaturation éthylénique, soit à insaturation acétylénique. L'additif de rhéologie polyamide obtenu peut être préparé à partir d'une poudre, ou en combinaison avec un solvant ou un plastifiant, pour former un liquide ou une pâte, ledit polyamide devant préalablement être dilué.

JP 2004107543 décrit un additif thixotrope sous forme de pâte exempte de grains, ladite pâte étant un produit de réaction entre un composant diamide et une résine époxy additionnée en fin de synthèse, ledit composant diamide étant modifié chimiquement pour pouvoir réagir sur la résine époxy.

D'autre part, JP 2002146336 décrit la préparation d'un additif de rhéologie sous forme de pâte, obtenu par mélange de diamides d'acide gras, dispersés dans un solvant à base d'alcool, d'un composé de type cycloaliphatique et d'un ester. La dispersion ainsi obtenue est ensuite soumise à un traitement thermique. La pâte finale ne contient pas de plastifiant ; elle est ensuite utilisée comme agent thixotrope dans des résines polyester insaturées.

D'autres additifs de rhéologie peuvent être utilisés pour augmenter la viscosité de systèmes mastics ou adhésifs en phase solvant ou sans solvant. Parmi ceux-ci, on peut citer les poudres de polyamides, les poudres de dérivés à base d'huile de ricin hydrogénée, les silices pyrogénées, les carbonates de calcium précipités ou broyés. Les silices pyrogénées et les carbonates de calcium sont de nature minérale et nécessitent une dispersion du mélange à très haute vitesse. Toutefois, ces charges minérales présentent des problèmes de stabilité et de sédimentation dans le temps, avec des effets résultants négatifs sur les propriétés mécaniques du système final. Un autre inconvénient particulier des poudres de polyamide et de dérivés de l'huile de ricin hydrogénée est la nécessité d'activation du système lors de la fabrication de la composition d'application finale pour l'utilisateur (formulateur). Cette activation requiert un cisaillement à haute vitesse, et un chauffage correspondant à des montées de température allant jusqu'à près de 120°C selon les produits, ainsi qu'une durée minimale nécessaire, dépendante des conditions de température et du système, pour développer des propriétés rhéologiques finales optimales. Ces additifs confèrent à la composition à laquelle ils sont incorporés un comportement thixotrope caractérisé par une rhéofluidification marquée, c'est-à-dire une réduction de la viscosité lorsque le cisaillement augmente, puis une reprise en viscosité dépendante du temps (équivalent à un effet d'hystérésis). Ce type d'additifs apporte à la composition finale d'excellentes propriétés d'application qui se caractérisent par une forte viscosité au repos, une bonne stabilité de cette viscosité au stockage, une bonne anti-sédimentation, une facilité d'application et d'extrusion et une bonne résistance à la coulure une fois appliquée. Malgré le fait que ce type d'additifs apportent de bonnes propriétés à la composition finale, il présente comme principal inconvénient le fait d'avoir besoin d'une phase d'activation, parfois difficile à contrôler, difficilement reproductible par l'utilisateur final, et coûteuse en temps et en énergie. JP 63015876 décrit une composition thixotrope de prépolymère polyuréthane ayant des groupements terminaux isocyanates, comprenant comme agent thixotrope un diamide d'acide gras à base d'un mélange d'huile de ricin hydrogénée et d'un acide gras saturé en C₁₄-C₁₈ et d'éthylène diamine ou de butane diamine 1,4, le taux dudit diamide étant de 1 à 8% en présence de 5-50% de charges, de 1-20% de plastifiant et d'autres additifs. US 5,373,042 décrit un composé de moulage et d'étanchéité monocomposant à base de prépolymères silylés, comprenant au moins un substituant hydrolysable sur l'atome de Si, des composés organométalliques à base d'étain comme catalyseur, des charges minérales et du PVC et un amide d'acide gras comme agent thixotrope. US 4,462,926 décrit des agents thixotropes convenables pour des résines polyesters insaturés, lesquels agents consistent au moins en un amide cyclohéxyle, un acide gras insaturé supérieur et un autre composant, oligomère ester amide.

Le problème technique de l'invention, par rapport à l'état de l'art antérieur cité, consisite donc en la mise au point d'un produit amélioré présentant des propriétés comparables à celles apportées par les poudres de polyamide et les dérivés de l'huile de ricin hydrogénée, sans nécessiter de phase d'activation par l'utilisateur final, ledit produit étant par conséquent plus facile et plus rapide à mettre en oeuvre par l'utilisateur final, avec d'autres avantages particuliers liés à la résolution de ce problème général. le terme " sous forma de pâte pré-activée" doit être considéré comme signifiant que l'additif est prêt à l'emploi par l'utilisateur final (formulateur de mastics, de colles, d'adhésifs, ou de revêtements tels que peintures, ou vernis, ou gek coats, ou encres, ou de composition de moulage), par simple mélange dans la formulation finale d'application, sans avoir besoin d'aucune activation spécifique in situ dans cette formulation (conditions spécifiques de température, de cisaillement et de durée à respecter).

Plus particulièrement, ces avantages liés à la résolution du problème technique général par rapport à l'état de l'art sont les suivants :
- faibles émissions de composés organiques volatiles (COV) et moindre risque d'inflammation ou d'explosion,
- absence de poudres pulvérulentes à manipuler par l'utilisateur final, ce qui évite la formation de poussières dans l'atmosphère et sur le lieu de fabrication des compositions finales, et par conséquent la nécessité de ventilation, pour éviter la respiration de ces poussières,
- possibilité de manipuler en l'absence de tout solvant dans la formule finale, l'additif pouvant être utilisé aussi bien dans des compositions solvantées d'application que dans des compositions sans solvants,
- flexibilité d'utilisation et de manipulation, la pâte pré-activée étant facile à disperser au sein de la composition, soit en même temps que les charges en début de fabrication, soit en fin de fabrication, tout en conservant des propriétés rhéologiques au moins comparables à celles obtenues à partir de poudres de type polyamides, diamides ou huiles de ricin selon l'état de l'art antérieur,
- enfin et surtout, pas besoin de l'étape d'activation, la mise en oeuvre de l'additif selon l'invention ne nécessitant ni chauffage, ni haut cisaillement, ni temps d'activation, ce qui permet un gain :
   ■ en temps de production, puisqu'il n'y a pas d'étape de chauffage, et que l'étape de refroidissement est réduite, voire supprimée, lors de la préparation de la composition finale,
   ■ en énergie, due à l'absence de chauffage et de refroidissement,
   ■ en productivité pour l'utilisateur final,
   ■ en reproductibilité et fiabilité des performances finales.

Ainsi, le premier objet de l'invention est un additif de rhéologie sous forme de pâte pré-activée comprenant :
A) au moins un diamide d'acide gras, introduit sous forme de poudre, ladite poudre pouvant optionnellement contenir de l'huile de ricin hydrogénée, en plus dudit diamide,
B) au moins un plastifiant organique, ledit plastifiant étant liquide à température ambiante.

Le deuxième objet de l'invention est un procédé de préparation de tels additifs de rhéologie par dispersion et activation par chauffage contrôlé.

L'invention couvre également une composition comprenant un liant organique et un additif de rhéologie selon la présente invention, ainsi que le procédé de préparation d'une telle composition.

Un autre objet de l'invention est l'utilisation de l'additif de rhéologie ou de la composition selon l'invention pour des applications de revêtements de peintures, vernis, encres, gel coats, plastisols à base de PVC, ou pour des applications de mastics, colles, adhésifs, agents d'étanchéité, compositions de moulage, ou encore pour des applications cosmétiques.

Enfin, un dernier objet de l'invention concerne les articles finis tels que les revêtements, joints d'étanchéité, composites ou pièces moulées, ou cosmétiques obtenus à partir d'un tel additif ou d'une telle composition selon l'invention.

Le premier objet de l'invention est donc un additif de rhéologie sous forme de pâte pré-activée comprenant :
A) au moins un diamide d'acide gras, introduit sous forme de poudre, ladite poudre pouvant optionnellement contenir, en plus dudit diamide, de l'huile de ricin hydrogénée,
B) au moins un plastifiant organique, ledit plastifiant étant liquide à température ambiante.

De préférence, ledit plastifiant est un plastifiant organique polaire comportant au moins un groupement polaire, de préférence un groupement éther et/ou un groupement ester et/ou un groupement époxy.

Ledit plastifiant peut d'abord comporter au moins un groupement éther, et dans ce cas il peut être choisi parmi les polyéthers tels que homopolymères et/ou copolymères, d'oxyde d'éthylène et/ou d'oxyde de propylène et/ou un mélange desdits polyéthers (homopolymères et/ou copolymères) et/ou leurs dérivés, ces dérivés comprenant entre autre lesdits polyéthers bloqués en bout de chaîne par un groupement alkoxy en C₁ (méthoxy) à C₄ (butoxy), ou par un groupement ester en C₂ (acétate) à C₄ (butyrate),
lesdits polyéthers ayant une masse moléculaire moyenne en poids Mw allant de 150 à 6000, et de préférence de 1000 à 3000. Le terme « copolymère » est à interpréter comme comprenant aussi bien les copolymères statistiques ou séquencés.

Ledit plastifiant peut ensuite comporter au moins un groupement ester, et il peut être choisi parmi les monoesters et/ou les polyesters (esters multifonctionnels), obtenus à partir d'alcools en C₄ à C₂₁, alcools éventuellement alkoxylés par exemple avec 1 à 10 unités alkoxy choisies parmi les motifs oxyéthylène (OE) et/ou oxypropylène (OP), et à partir de mono- ou polyacides de fonctionnalité allant de 1 à 4, sélectionnés parmi :
- les acides organiques choisis parmi les acides aromatiques ayant une longueur de chaîne (sans fonction -CO₂H allant de C₆ à C₁₀, et/ou les acides aliphatiques ayant une longueur de chaîne (sans fonction -CO₂H) allant de C₄ à C₁₈, ou
- les acides minéraux.

Les esters d'acides aromatiques peuvent être choisis parmi les esters phtaliques (phtalates) et triméllitiques (trimellitates). Les esters d'acides aliphatiques peuvent être choisis parmi les esters adipiques (adipates), citriques (citrates), sébaciques (sébacates), azélaïque (azélates). Les esters d'acides minéraux peuvent être choisis parmi les esters sulfoniques (sulfonates), notamment les sulfonates d'alcoyle en C₁₀ à C₂₁, sulfuriques (sulfates), sulfiniques (sulfinates), phosphoriques (phosphates), phosphoniques (phosphonates) et phosphiniques (phosphinates).

Enfin, ledit plastifiant peut aussi comporter au moins un groupement époxy, et peut être choisi parmi les huiles époxydées à base d'acide gras, dont la longueur de chaîne peut aller de C₁₆ à C₁₈, telles que l'huile de soja époxydée.

De manière préférée, dans ledit additif de rhéologie il y a absence de tout autre composé, sélectionné parmi les alcools de masse moléculaire Mw < 150, comme le méthanol, l'éthanol, le propanol, le butanol ou l'alcool benzylique, ou sélectionné parmi les solvants aprotiques polaires comme la N-méthylpyrrolidone, la N-éthylpyrrolidone, la N-butylpyrrolidone, l'acétonitrile, la N,N-diméthylformamide, le N,N'-dimétylacétamide, le diméthylsulfoxyde, la N,N,N',N'-tétraméthylurée, l'hexaméthylphosphoramide, le triamide d'hexaméthylphosphoreux ou le carbonate de propylène.

Parmi les plastifiants préférés, on peut citer ceux comportant au moins un groupement ester d'acide aromatique en C₆ à C₁₀, en particulier les plastifiants sélectionnés parmi les mono- et/ou les di-alkylphtalates, et encore plus préférentiellement parmi les di-alkylphtalates, avec lesdits alkyles pouvant être identiques ou différents, et choisis parmi les alkyles en C₇ à C₁₈, et de préférence en C₁₀ à C₁₂.

Dans cette famille de plastifiants (di-alkylphtalates), le plus préféré est le di-iso-undécylphtalate. Dans la famille des polyéthers, les plus préférés sont les polyéthers homopolymères d'oxyde de propylène (polypropylène glycols) de masse moléculaire moyenne en poids, Mw allant de 1000 à 3000, et plus particulièrement le polypropylène glycol (PPG) de Mw égale à 2000, et/ou leurs dérivés, choisis parmi les monoesters, de préference en C₂ à C₄) ou les monoéthers en C₁ à C₄, tels que les dérivés monométhoxylés ou monoéthoxylés.

Ledit plastifiant peut avoir une température d'ébullition supérieure à 200°C, et de préférence supérieure à 250°C (à pression atmosphérique). Il doit être liquide à température ambiante pour que ledit diamide soit dispersible dans ledit plastifiant.

Ledit diamide d'acide gras introduit sous forme de poudre a une granulométrie inférieure à 100 µm, et de préférence inférieure à 50 µm, et plus préférentiellement au moins 90% dudit diamide a une granulométrie inférieure à 20 µm, et de préférence inférieure à 15 µm.

Le taux en poids du diamide d'acide gras peut varier de 10 à 40%, et de préférence de 15 à 30%.

Un diamide d'acide gras, convenable pour l'invention, peut être obtenu par polycondensation entre au moins une diamine primaire en C₂ à C₁₂ et au moins un acide monocarboxylique de longueur de chaîne en C₃ à C₂₂, le produit de réaction pouvant optionnellement être dilué dans de l'huile de ricin hydrogénée, et dans ce cas à un taux variant de 10 à 100% en poids par rapport au total diamide + huile de ricin hydrogénée, et de préférence à un taux variant de 20 à 100% en poids. L'huile de ricin hydrogénée peut être utilisée pour adapter l'affinité du mélange final (diamide + huile de ricin hydrogénée) par rapport à la composition de la formulation finale en application.

Dans le cas où le diamide est dilué dans l'huile de ricin hydrogénée, l'addition se fait à une température comprise entre 140 et 220°C. A la fin de l'addition, on obtient une masse solide qui est broyée.

Selon un mode de réalisation préféré de l'invention, l'additif de rhéologie selon l'invention est susceptible d'être obtenu par un procédé spécifique, tel que décrit ci-dessous.

Le procédé spécifique de préparation dudit additif de rhéologie, qui est le deuxième objet de l'invention, comprend les étapes suivantes :
i) dispersion progressive dudit diamide sous forme de poudre, dans ledit plastifiant jusqu'à obtention d'une dispersion homogène, à température ambiante contrôlée, grâce à une régulation de la température, ledit diamide et ledit plastifiant étant tels que définis précédemment,
ii) maintien de la dispersion homogène obtenue lors de l'étape i) en au moins un isotherme avec une température correspondante allant de 50 à 120°C, et de préférence allant de 60 à 100°C, pendant une durée de 1 à 100 heures, et de préférence de 15 à 80 heures.

L'homogénéité de la dispersion se caractérise par une absence de grains, lors de l'application du film de ladite dispersion entre deux plaques en verre (test des plaques de verre). Pour obtenir cette homogénéité, ledit diamide est dispersé dans ledit plastifiant à une vitesse de cisaillement comprise de préférence entre 2 et 6 m.s⁻¹, pour une préparation à l'échelle du laboratoire sur une capacité n'excédant pas 1 litre.

La fin de l'étape ii), dite aussi « étape d'activation ou de maturation», est caractérisée par une valeur de pénétration maximale de la pâte formée inférieure à 10 mm, et de préférence inférieure à 5 mm, mesurée selon la norme ASTM D 217. Cette valeur de pénétration maximale est à associer a une méthode de référence de préparation de ladite pâte pré-activée, qui correspond à une préparation à l'échelle du laboratoire sur une capacité n'excédant pas 1 litre, l'échantillon étant ainsi parfaitement homogène. La méthode de préparation de ladite pâte pré-activée est décrite ci-dessous dans les exemples selon l'invention.

Le troisième objet de l'invention concerne une composition de liant organique comprenant en plus dudit liant au moins un additif de rhéologie tel que défini selon l'invention ou obtenu selon le procédé tel que défini selon l'invention, ladite composition pouvant être une composition de revêtement, cette dernière pouvant être utilisée comme revêtement de protection et/ou de décoration et/ou de traitement de surface de divers substrats, revêtement sélectionné parmi les peintures, vernis, gel coats pigmentés ou non pigmentés, encres, plastisols à base de PVC, ou une composition de mastic, de colle, d'adhésif, d'agent d'étanchéité, ou encore une composition de moulage pour composites ou pour pièces moulées, type SMC ou BMC ou stratifiées, types coques de bateaux ou panneaux en composites, ou pièces moulées par coulée, avec application de la composition par projection au pistolet ou à la brosse ou au rouleau, ou enfin une composition de cosmétique.

Ledit additif de rhéologie selon l'invention, tel que décrit ci-dessus, peut être présent dans ladite composition de liant organique, a une teneur en poids variant de 1 à 30%, et de préférence de 2 à 25%, et ledit diamide peut être présent en tant que matière active sèche à une teneur en poids variant de 0,2 à 8%, et de préférence de 1 à 6%, par rapport au poids de ladite composition de liant organique.

La composition de liant organique selon l'invention peut comprendre d'autres composants comme par exemple des charges, des plastifiants, des agents mouillants ou encore des pigments.

Selon un mode de réalisation plus particulier, la composition selon l'invention est réticulable, soit thermiquement, soit par irradiation sous rayonnements tels qu'UV (en présence d'au moins un photo-amorceur) et/ou EB (faisceau d'électrons, sans amorceur), y compris auto-réticulable à température ambiante, ou elle est non réticulable. La composition peut être réticulable mono-composante (un seul composant réactif) ou bi-composante (liant à base de deux composants réactifs entre eux par mélange lors de l'utilisation).

Ledit liant organique peut être sélectionné parmi au moins un système réactif époxy-amine (bi-composant réticulable), un polyester insaturé, un vinylester, une résine époxydée, une résine silicone réactive, une alkyde greffée par un polyester ou un polyamide ou de type diurée-diuréthane, ou alkyde non greffée, un polyuréthane ou une silicone, un polyuréthane bi-composant réticulable, un polysiloxane, un polymère polysulfure, un polymère acrylique réactif, un oligomère multifonctionnel (méth)acrylate ou oligomère acrylique acrylé ou oligomère multifonctionnel allylique, un élastomère type SBR, polychloroprène ou caoutchouc butyle, ou un pré-polymère silané, de préférence un polyéther silané ou un polyuréthane silané, ou un polyéther-uréthane silané avec fonction -OH ou -CO₂H.

Dans un cas plus particulier, ledit liant organique peut être sélectionné parmi les systèmes réactifs bi-composants réticulables suivants : les systèmes époxy-amines ou époxy-polyamides comprenant au moins une résine époxy comportant au moins deux groupements époxy et au moins un composé aminé ou polyamide comportant au moins deux groupements amines, les systèmes polyuréthanes comprenant au moins un polyisocyanate et au moins un polyol, les systèmes polyol-mélamines, et les systèmes polyesters à base d'au moins un époxy ou d'un polyol réactif avec au moins un acide ou un anhydride correspondant.

Selon d'autres cas particuliers, ledit liant organique peut être un système bi-composant polyuréthane réticulable ou un système bi-composant réticulable polyester à partir d'un système de réaction époxy-acide ou anhydride carboxylique, ou d'un système polyol-acide ou anhydride carboxylique, ou un système de réaction polyol-mélamine dans lequel le polyol est une résine acrylique hydroxylée, ou un polyester ou un polyéther polyol.

Selon une variante, la composition selon l'invention est une composition de mastic, de colle, d'adhésif ou d'agent d'étanchéité, qui est auto-réticulable, et à base de polyéther-silane ou de polyuréthane-silane.

Plus particulièrement, la composition selon l'invention peut être une composition de mastic mono-composant à base de pré-polymère silylé (ou silané, ce terme étant à considérer comme synonyme de silylé pour la présente invention), et de préférence de polyéther silylé ou de polyuréthane silylé (polyéther-uréthane silylé), comme les mastics KANEKA MS POLYMER^{™} et KANEKA SILYL^{™}. Les MS polymères peuvent être utilisés dans :
- la construction et le bâtiment pour les joints de dilatation, le montage de vitrage, les joints de menuiserie, les joints de maçonnerie, les préfabriqués (panneaux sandwich ou camion frigorifique, par exemples), les joints pour parquets,
- l'industrie pour la marine, l'automobile (carrosserie industrielle, joints de pare-brise),
- la grande distribution pour les mastics et adhésifs, avec et sans solvant,
- le génie civil.

D'autres composants peuvent être ajoutés ou substitués aux compositions de mastic mono-composant à base de pré-polymère silylé, comme d'autres types de liants, des pigments colorés, divers plastifiants, des charges de type carbonates de calcium précipités ou broyés, des dérivés de glycéride, des silices, comme des silices pyrogénées, d'autres additifs tels que les UVA (anti-oxydants UV), comme le 2,4-diterbutyl-6-(5-chlorobenzotriazole-2-yl)phénol (Tinuvin^{®} 327 de Ciba), les stabilisants lumière à base d'amines stériquement encombrées, tels que les HALS (Hindered Amine Light Stabilizers), comme le bis(2,2,6,6-tétraméthyl-4-pipéridyl)sébacate (Tinuvin^{®} 770 de Ciba), les cires, et d'autres types de catalyseurs, comme les sels d'étain.

L'invention comprend aussi un procédé de préparation de ladite composition de liant organique selon l'invention, ledit procédé comprenant les étapes suivantes :
i) addition de l'additif selon l'invention dans ladite composition de liant organique,
ii) homogénéisation du mélange avec un malaxeur et/ou mélangeur planétaire et/ou disperseur à haute vitesse, sans avoir aucun besoin, à cette étape, d'appliquer un traitement thermique d'activation.
Le terme « haute vitesse » signifie des vitesses tangentielles allant de 2 à 15 m.s⁻¹.

Un autre objet selon l'invention concerne l'utilisation d'au moins un additif de rhéologie ou d'une composition selon la présente invention dans des applications de revêtements, tels que les revêtements de peintures, de vernis, d'encres, de gels coats pigmentés ou non pigmentés, ou de plastisols à base de PVC, ou dans des applications de mastics, de colles, d'adhésifs, d'agents d'étanchéité, de compositions de moulage pour composites, de pièces moulées, de stratifiés SMC/BMC, ou dans des applications cosmétiques, comme les vernis à ongles.

Enfin, le dernier objet de l'invention concerne les objets finis tels que les revêtements obtenus à partir de l'utilisation d'au moins un additif de rhéologie ou d'une composition selon la présente invention, ou par l'utilisation des procédés de préparation respectifs, lesdits revêtements étant sélectionnés parmi les peintures, ou les vernis, ou les encres, ou les gel coats pigmentés ou non pigmentés, ou les plastisols à base de PVC, ou les mastics, ou les colles, ou les adhésifs, ou les joints d'étanchéité, ou les composites, ou les pièces moulées, ou les stratifiés SMC/BMC résultant des compositions de moulage, ou encore les produits cosmétiques, comme les vernis à ongles

A titre d'illustration de l'invention, les exemples suivants démontrent sans aucune limitation, les performances de l'additif de rhéologie selon la présente invention.

### I- Matières premières utilisées

**Tableau 1**

| | Fonction | Référence commerciale | Fournisseur |
|---|---|---|---|
| Polyéther silylé | Liant | MS POLYMER^{™} S203H | Kaneka |
| Polyéther silylé | Liant | MS POLYMER^{™} S303H | Kaneka |
| Di-iso-undécylphtalate (DIUP) | Plastifiant | Jayflex^{®} DIUP | Exxon Mobil |
| Polyamide | Additif de rhéologie | Crayvallac^{®} SL | Cray Valley |
| Carbonate de calcium | Charges | Carbital^{®} C11OS | Imerys |
| Dioxyde de titane | Pigment | RL 90 | Millenium |
| 2,4-diterbutyl-6-(5-chlorobenzotriazole-2-yl) phénol | Stabilisant UV | Tinuvin^{®} 327 | Ciba |
| Bis(2,2,6,6-tétraméthyl-4-pipéridyl)sébacate | Stabilisant lumière | Tinuvin^{®} 770 | Ciba |
| Silane | Agent déshydratant | Dynasilan^{®} VTMO | Degussa Sivento |
| Silane | Promoteur d'adhérence | Dynasilan^{®} DAMO | Degussa Sivento |
| Sel d'étain | Catalyseur | Métatyn^{®} KAT 740 | Acima |

### II - Préparation des additifs de rhéologie : diamide d'acide gras et pâte pré-activée

### Préparation du diamide d'acide gras :

450 g d'acide 12-hydroxystéarique et 430 g d'acide caprique sont chauffées à 80°C. 120 g d'éthylène diamine sont ensuite chargées. L'amine réagit immédiatement avec les acides pour former les sels correspondants. Il en résulte une réaction exothermique qui fait progresser la température au sein du réacteur de 80 à 130°C. Le réacteur est ensuite chauffé rapidement jusqu'à ce que la température du batch atteigne 200°C. Le réacteur est ensuite maintenu à cette température, jusqu'à ce que les indices d'acide et d'amine soient inférieurs à 6 mg KOH/g.

Le contenu du réacteur est ensuite déchargé, refroidi, puis finement broyé.

### Préparation de la pâte pré-activée :

### a) Plastifiant : DIUP

250 g du diamide d'acide gras préalablement broyé et 750 g de di-iso-undécylphtalate (DIUP) sont chargées dans une boîte métallique d'1 litre (hauteur : 13 cm, diamètre : 11 cm), à température ambiante. En utilisant un disperseur Dispermat^{®} CV muni d'une pâle de 4 cm de diamètre, les 2 produits sont mélangés à 1500 tours/min pendant 15 min, à une température n'excédant pas 20°C, par régulation de la température par circulation d'eau froide. La boîte est ensuite refermée soigneusement et introduite dans une étuve préalablement préchauffée à 70°C pendant 24 heures.

Le produit final est une pâte souple, blanche, caractérisée par un taux de matière active sèche de 25%, et une résistance à la pénétration mesurée selon la norme ASTM D 217 de 2,00 mm.

### b) Plastifiant : PPG 2000

250 g du diamide d'acide gras préalablement broyé et 750 g de polypropylène glycol de masse 2000 (PPG 2000) sont chargées dans une boîte métallique de 1 litre (hauteur : 13 cm, diamètre : 11 cm), à température ambiante. En utilisant un disperseur Dispermat^{®} CV muni d'une pâle de 4 cm de diamètre, les deux produits sont mélangés à 1500 tours/min pendant 15 min, à une température n'excédant pas 20°C, par régulation de la température par circulation d'eau froide. La boîte est ensuite refermée soigneusement et introduite dans une étuve préalablement préchauffée à 65°C pendant 24 heures.

Le produit final est une pâte souple, blanche, caractérisée par un taux de matière active sèche de 25%, et une résistance à la pénétration mesurée selon la norme ASTM D 217 de 3,11 mm.

### III - Utilisation des additifs de rhéologie pour la préparation de mastics

L'additif de rhéologie sous forme de pâte pré-activée est utilisé dans la préparation des mastic de type MS polymères.

Le stockage de l'additif de rhéologie se fait en l'absence d'eau et d'humidité pour éviter tout début de polymérisation par réticulation. La réaction de réticulation (auto-réticulation) se produit au moment de l'utilisation, en présence de l'humidité ambiante, à humidité relative (HR) > 50%.

### Fabrication des mastics :

Les mastics sont fabriqués au laboratoire avec un appareil de type Molteni LABMAX disperseur D2 (série 2000). Cet équipement permet de reproduire les conditions industrielles mais à petite échelle (pilote). Il est muni d'un disperseur planétaire de 65 mm de diamètre, d'une racle et d'une pompe à vide qui exclut l'entrée d'humidité pendant la fabrication. Cet appareil permet la fabrication de 1500 g de produit, ce qui équivaut à trois cartouches. Les cartouches utilisées sont des cartouches Fischbach E310 HPDE, 310 ml - accessoires KO1 - DO1A0.

Les MS polymères et le plastifiant DIUP sont mélangés à 1000 tours/min, à une vitesse tangentielle de 3,5 m.s⁻¹, pendant 5 minutes. L'additif de rhéologie est ensuite ajouté et dispersé à 2600 tours/min (8,8 m.s⁻¹), le mélange étant maintenu à une température de 30°C par circulation d'eau.

Les stabilisants lumière et UV, le dioxyde de titane, ainsi que le carbonate de calcium ayant été préalablement séché pendant 12 heures à 90°C, sont incorporés puis dispersés sous vide à 2600 tours/min. Les charges sont complètement dispersées au bout d'une heure environ.

### IV - Méthodes de caractérisation

Le comportement rhéologique des compositions comprenant au moins un additif selon la présente invention a été caractérisé selon les tests suivants :

### 1) Extrudabilité ou grammage

### 1-1) Principe

Cette méthode est spécifique à la détermination de l'extrudabilité d'un mastic mono-composant, à partir de l'emballage dans lequel il a été conditionné, le mastic mono-composant étant extrudé sous pression d'air comprimé.

L'extrudabilité est exprimée en un poids extrudé sur un temps défini (g.min⁻¹).

### 1-2) Appareillage

L'appareillage utilisé est le suivant :
- un pistolet pneumatique pour l'application du mastic sur chantier,
- un compresseur à air avec une valve et un manomètre pour maintenir l'apport d'air comprimé à 250 ± 10 KPa (soit 2,5 bars ± 0,1 bar),
- un cylindre en plastique de 500 ml,
- un chronomètre étalonné en secondes,
- une buse d'extrusion avec un orifice de diamètre de 5 ± 0,3 mm,
- une balance de précision pour peser l'échantillon pendant l'extrusion.

### 1-3) Conditionnement des produits

Les produits à tester sont conditionnés pendant 24 heures dans un laboratoire climatisé à 23°C et 50% HR, avant le début de l'essai.

### 1-4) Préparation des produits

Le bout de la douille filetée d'une cartouche (cartouche Fischbach E310 : HPDE, 310 ml - accessoires KO1 - DO1A0) doit être coupé pour former l'orifice le plus large possible, d'un diamètre de 6 mm au moins. La buse d'extrusion est alors vissée sur la cartouche.

### 1-5) Mode opératoire de caractérisation rhéologique

L'essai est réalisé à la température du laboratoire climatisé (23°C et 50% HR). Trois essais sont réalisés sur une même cartouche.

L'emballage, préparé comme précédemment, est placé dans le pistolet pneumatique, puis l'alimentation en air comprimé est augmenté jusqu'à 250 KPa.

Le mastic est extrudé de l'emballage dans la buse, pour la remplir complètement, et ainsi dégager tout air éventuellement enfermé dans l'emballage. Le pistolet est ensuite placé verticalement au dessus du cylindre lui même posé sur la balance.

L'extrusion et le chronomètre sont mis en marche simultanément.

Environ 100 g de mastic sont extrudés.

De nouveau en simultané, l'extrusion et le chronomètre sont arrêtés.

L'essai est répété deux fois. Pour chaque essai le débit de l'extrusion est calculé, en g.min⁻¹, à partir du poids de mastic extrudé en fonction du temps d'extrusion.

### 2) Viscosité

Evaluation de la viscosité Brookfield^{®} à 23°C et 50% HR, avec une aiguille en T n° 95, et à trois vitesses différentes : 1, 5 et 10 tours/min (ou rpm), sur un viscosimètre Brookfield^{®}, avec système Hélipath^{™}, selon la norme EN ISO 2555. La viscosité Brookfield^{®} est exprimée en mPa.s. Le résultat est l'expression d'une moyenne de six valeurs.

### 3) Indice de thixotropie

Un indice de thixotropie (V1/V10) égale au ratio de la viscosité Brookfield^{®} obtenue à 1 tour/min sur la viscosité Brookfield^{®} obtenue à 10 tours/min est calculé.

Cette valeur relative et indicative du comportement thixotrope, est couramment utilisée dans le milieu industriel.

### 4) Consistance

La consistance des pâtes est mesurée grâce à un appareil de texture, selon la norme ASTM D 217. La norme se réfère à un appareil de mesure manuelle normalisé et a été reproduit par un analyseur de texture TAXT2i fabriqué par Thermorheo (fournisseur : Swantech). Le principe de la norme a été automatisée sur le TAXT2i de « Stable Micro System ».

### 4-1) Principe

Un cône adapté tel que décrit dans la norme ASTM D 217 est utilisé, avec une force donnée pour fournir une valeur de pénétration dans la pâte. Cette mesure « d'enfoncement » est exprimée en millimètres (mm).

La résistance à la pénétration augmente avec la consistance, et la valeur de pénétration diminue avec cette consistance.

### 4-2) Appareillage

L'appareillage utilisé est le suivant :
- un texturomètre automatique : TAXT2i,
- un cône dont la force appliquée est équivalente à son poids de 47,5 g, avec une aiguille formant un angle de 30°.

### 4-3) Conditionnement des produits

Les produits à tester doivent être conditionnés dans un laboratoire climatisé, dans des conditions de température et d'humidité de 23 ± 1°C et 50 ± 5% d'HR, pendant une durée de 24 heures au moins avant le début de l'essai.

### 4-4) Préparation des produits

Les produits sont conditionnés après la synthèse, dans des boîtes métalliques de 1 litre (hauteur : 13 cm, diamètre : 11 cm).

La mesure de texture est effectuée après le stade de maturation, et avant l'utilisation des échantillons.

### 4-5) Mode opératoire

L'essai est réalisé à la température du laboratoire climatisé. Trois essais sont réalisés sur un pot.

L'échantillon est posé sous le cône, et la pointe de l'aiguille est posée à la surface de la pâte.

Le programme sur le TAXT2i est ensuite mis en route, et la pénétration est déclenchée.

La valeur du déplacement de l'aiguille dans la pâte est ensuite relevée.

Les résultats sont exprimés en millimètres (mm).

### V - Exemples

Quatre formules de mastics différentes ont été réalisées :
- Exemple 1 : mastic sans additif,
- Exemple 2 : mastic avec un additif poudre Crayvallac^{®} SL,
- Exemple 3 : mastic avec une pâte pré-activée dans le DIUP,
- Exemple 4 : mastic avec une pâte pré-activée dans le PPG 2000.

Les 4 exemples ont été réalisés selon la formule suivante :

**Tableau 2**

| **Références matières premières** | **Nature** | **Ex. 1** | **Ex. 2** | **Ex. 3** | **Ex. 4** |
|---|---|---|---|---|---|
| MS POLYMER^{™} S203H | Polyéther silylé | 18 | 18 | 18 | 18 |
| MS POLYMER^{™} S303H | Polyéther silylé | 12 | 12 | 12 | 12 |
| Jayflex^{®} DIUP | Di-iso-undécyLphtalate (DIUP) | 15 | 15 | 3,6 | 3,6 |
| Crayvallac^{®} SL | Polyamide | 0 | 3,8 | 15,2* | 15,2* |
| Carbital^{®} C 110S | Carbonate de calcium | 46,1 | 46,1 | 46,1 | 46,1 |
| RL 90 | Dioxyde de titane | 2,0 | 2,0 | 2,0 | 2,0 |
| Tinuvin^{®} 327 | 2,4-diterbutyl-6-(5-chlorobenzotriazole-2-yl)phénol | 0,3 | 0,3 | 0,3 | 0,3 |
| Tinuvin^{®} 770 | Bis(2,2,6,6-tétraméthyl-4-pipéridyl)sébacate | 0,3 | 0,3 | 0,3 | 0,3 |
| Dynasilan^{®} VTMO | Silane | 1 | 1 | 1 | 1 |
| Dynasilan^{®} DAMO | Silane | 0,8 | 0,8 | 0,8 | 0,8 |
| Métatyn^{®} KAT 740 | Sel d'étain | 0,7 | 0,7 | 0,7 | 0,7 |
| Total | | 96,2 | 100,0 | 100,0 | 100,0 |

| | | | | | |
|---|---|---|---|---|---|
| * dans le plastifiant (25% en poids de polyamide dans 75% en poids de plastifiant). | | | | | |

### Exemple 1 (comparatif) : mastic sans additif de rhéologie

La dispersion se fait pendant une heure à 2600 tours/min (8,8 m.s⁻¹), en adiabatique à l'ambiante.

### Exemple 2 (comparatif) : mastic avec un additif poudre Crayvallac^{®} SL

La dispersion est chauffée pendant deux heures à 80°C à 2600 tours/min pour activer l'additif poudre ayant une granulométrie maximale de 15 µm.

A la fin de l'activation, au bout de deux heures, la vitesse du Molteni est abaissée à 1500 tours/min (5,1 m.s⁻¹) et le mélange est refroidi par circulation d'eau froide pour atteindre une température inférieure à 50°C.

### Exemples 3 et 4 (invention) : mastic avec une pâte pré-activée respectivement dans le DIUP et dans le PPG 2000 (pâtes pré-activées décrites précédemment aux § II a) et b))

La dispersion se fait pendant une heure à 2600 tours/min, sans chauffer. L'addition du triméthoxysilane de n-(2-aminoéthyl-3-amino)propyle (Dynasylan^{®} DAMO) et de vinyltriméthoxysilane (Dynasylan^{®} VTMO) se fait à une température inférieure à 50°C pour éviter leur évaporation. Quand la température est inférieure à 50°C, le promoteur d'adhérence Dynasylan^{®} DAMO, ainsi que l'agent déshydratant Dynasylan^{®} VTMO, sont ajoutés et mélangés sous vide et sous faible agitation à 1000 tours/min pendant 15 min.

Le catalyseur est ensuite ajouté et mélangé sous vide pendant 10 min à 1000 tours/min.

Le mastic est ensuite mis en cartouches, puis mis au repos pendant au moins 24 heures dans une salle climatisée à 23°C.

### VI - Résultats

**Tableau 3 : Consistance**

| Déplacement (pénétration) de l'aiguille dans la pâte pré-activée à base de DIUP (mm) | Déplacement (pénétration) de l'aiguille dans la pâte pré-activée à base de PPG 2000 (mm) |
|---|---|
| 2,00 | 3,11 |

**Tableau 4**

| Exemple | Additif de rhéologie | Temps de fabrication (min) | Aspect du mastic | Extrudabilité (g/min) | Viscosités (mPa.s) | | | Indice de thixotropie |
|---|---|---|---|---|---|---|---|---|
| | | | | | 1 rpm | 5 rpm | 10 rpm | |
| **Ex. 1** | Sans additif | 90 | bon | 1430 | 80000 | 40000 | 28000 | 2,9 |
| **Ex. 2** | Crayvallac^{®} SL | 260 | bon | 790 | 1130000 | 412000 | 259000 | 4,4 |
| **Ex. 3** | Pâte dans le DIUP | 90 | bon | 595 | 1460000 | 544000 | 348000 | 4,2 |
| **Ex. 4** | Pâte dans le PPG 2000 | 100 | bon | 581 | 1410000 | 548000 | 353000 | 4,0 |

### Figures :

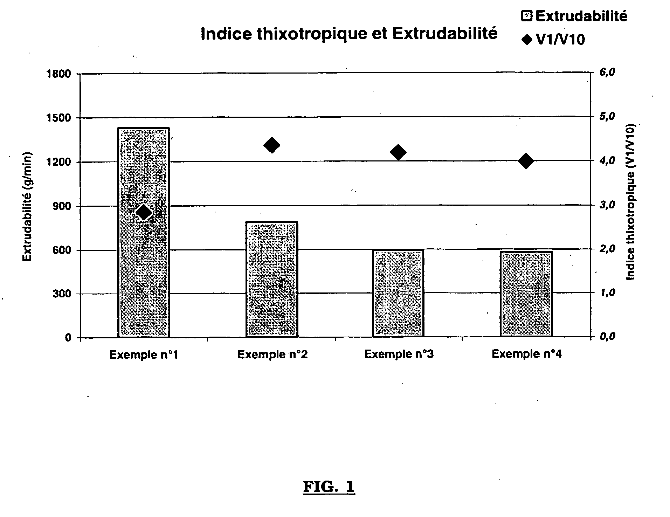

La figure 1 présente l'extrudabilité et l'indice de thixotropie (V1/V10) comparés, d'additifs de rhéologie sous différentes formes, comme décrit dans les exemples 1 à 4.

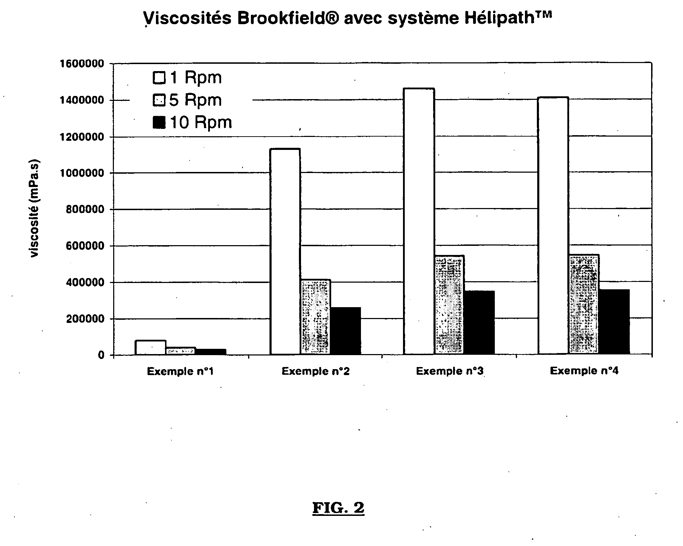

La figure 2 présente les viscosités Brookfield^{®} (avec système Hélipath^{™}) comparées, d'additifs de rhéologie sous différentes formes, comme décrit dans les exemples 1 à 4.

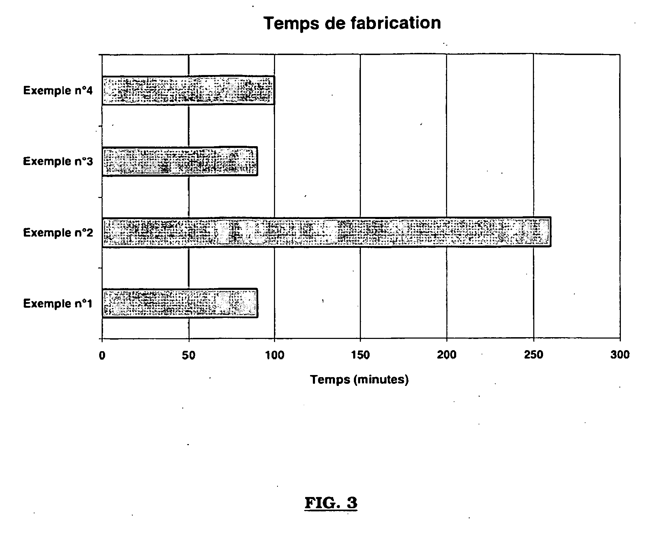

La figure 3 présente les temps de fabrication comparés, d'additifs de rhéologie sous différentes formes, comme décrit dans les exemples 1 à 4.

## Revendications

1. Additif de rhéologie sous forme de pâte pré-activée **caractérisé en ce qu'**il comprend :
A) au moins un diamide d'acide gras, introduit sous forme de poudre, ladite poudre pouvant optionnellement contenir, en plus dudit diamide, de l'huile de ricin hydrogénée,
B) au moins un plastifiant organique, ledit plastifiant étant liquide à température ambiante,
et **en ce que** le taux en poids dudit diamide d'acide gras varie de 10 à 40% et **en ce que** la valeur de pénétration maximale de ladite pâte est inférieure à 10 mm et de préférence inférieure à 5 mm mesurée selon la norme ASTM D217.

2. Additif selon la revendication 1 **caractérisé en ce que** ledit plastifiant est un plastifiant organique polaire, comportant au moins un groupement éther et/ou un groupement ester et/ou un groupement époxy.

3. Additif selon la revendication 2 **caractérisé en ce que** ledit plastifiant comporte au moins un groupement éther, et qu'il est choisi parmi les polyéthers homopolymères et/ou copolymères d'oxyde d'éthylène et/ou d'oxyde de propylène et/ou un mélange desdits polyéthers et/ou leurs dérivés, ces dérivés comprenant entre autre lesdits polyéthers bloqués en bout de chaîne par un groupement alkoxy en C₁ à C₄, ou par un groupement ester en C₂ à C₄, lesdits polyéthers ayant une masse moléculaire moyenne en poids Mw allant de 150 à 6000.

4. Additif selon la revendication 2 **caractérisé en ce que** ledit plastifiant comporte au moins un groupement ester, et qu'il est choisi parmi les monoesters et/ou polyesters, obtenus à partir d'alcools en C₄ à C₂₁, alcools éventuellement alkoxylés, et à partir de mono- ou polyacides de fonctionnalité allant de 1 à 4, sélectionnés parmi :
- les acides organiques choisis parmi les acides aromatiques ayant une longueur de chaîne allant de C₆ à C₁₀, et/ou les acides aliphatiques ayant une longueur de chaîne allant de C₄ à C₁₈, ou
- les acides minéraux.

5. Additif selon la revendication 4 **caractérisé en ce que** lesdits mono- et/ou polyesters sont des esters :
- d'acides aromatiques, choisis parmi les esters phtaliques et triméllitiques,
- d'acides aliphatiques, choisis parmi les esters adipiques, citriques, sébaciques, azélaïques, et
- d'acides minéraux, choisis parmi les esters sulfoniques, sulfuriques, sulfiniques, phosphoriques, phosphoniques et phosphiniques.

6. Additif selon la revendication 2 **caractérisé en ce que** ledit plastifiant comporte au moins un groupement époxy, choisi parmi les huiles époxydées à base d'acide gras.

7. Additif selon l'une des revendications 1 à 6 **caractérisé en ce que** ledit plastifiant est présent en l'absence de tout autre composé, sélectionné parmi les alcools de masse moléculaire Mw < 150, ou parmi les solvants aprotiques polaires choisis parmi: la N-méthylpyrrolidone, la N-éthylpyrrolidone, la N-butylpyrrolidone, l'acétonitrile, la N,N-diméthylformamide, le N,N'-diméthylacétamide, le diméthylsulfoxyde, la N,N,N',N'-tétraméthylurée, l'hexaméthylphosphoramide, le triamide d'hexaméthylphosphoreux ou le carbonate de propylène.

8. Additif selon la revendication 3 **caractérisé en ce que** ledit plastifiant est sélectionné parmi les polyéthers homopolymères d'oxyde de propylène, de masse moléculaire moyenne en poids Mw allant de 1000 à 3000, et/ou leurs dérivés.

9. Additif selon la revendication 5 **caractérisé en ce que** ledit plastifiant comporte au moins un groupement ester d'acide aromatique en C₆ à C₁₀, sélectionné parmi les mono- et/ ou les di-alkylphtalates.

10. Additif selon la revendication 9 **caractérisé en ce que** ledit plastifiant est au moins un di-alkylphtalate, avec lesdits alkyles étant identiques ou différents, et choisis parmi les alkyles en C₇ à C₁₈.

11. Additif selon la revendication 10 **caractérisé en ce que** ledit plastifiant est un di-iso-undécylphtalate.

12. Additif selon la revendication 5 **caractérisé en ce que** ledit plastifiant est un ester sulfonique sélectionné parmi les sulfonates d'alcoyle en C₁₀ à C₂₁.

13. Additif selon l'une des revendications 1 à 12 **caractérisé en ce que** ledit diamide d'acide gras est obtenu par polycondensation entre au moins une diamine primaire en C₂ à C₁₂ et au moins un acide monocarboxylique de longueur de chaîne en C₃ à C₂₂, le produit de réaction pouvant optionnellement être dilué dans de l'huile de ricin hydrogénée, et dans ce cas à un taux variant de 10 à 100% en poids par rapport au total diamide + huile de ricin hydrogénée.

14. Additif selon l'une des revendications 1 à 13 **caractérisé en ce qu'**il est susceptible d'être obtenu par un procédé comprenant les étapes suivantes :
i) dispersion progressive dudit diamide sous forme de poudre, dans ledit plastifiant jusqu'à obtention d'une dispersion homogène, à température ambiante contrôlée, grâce à une régulation de la température,
ii) maintien de la dispersion homogène obtenue lors de l'étape i) en au moins un isotherme avec une température correspondante allant de 50 à 120°C, pendant une durée de 1 à 100 heures.

15. Procédé de préparation de l'additif tel que défini selon l'une des revendications 1 à 14 **caractérisé en ce qu'**il comprend les étapes suivantes :
i) dispersion progressive dudit diamide sous forme de poudre, dans le plastifiant jusqu'à obtention d'une dispersion homogène, à température ambiante contrôlée, grâce à une régulation de la température, le diamide et le plastifiant étant tels que définis selon l'une des revendications 1 à 14,
ii) maintien de la dispersion homogène obtenue lors de l'étape i) en au moins un isotherme avec une température correspondante allant de 50 à 120°C, pendant une durée de 1 à 100 heures.

16. Procédé selon la revendication 15 **caractérisé en ce qu'**à la fin de l'étape ii) la valeur de pénétration maximale de la pâte formée est inférieure à 10 mm, mesurée selon la norme ASTM D 217.

17. Composition de liant organique **caractérisée en ce qu'**elle comprend en plus dudit liant au moins un additif tel que défini selon l'une des revendications 1 à 14, ou obtenu par un procédé tel que défini selon l'une des revendications 15 ou 16.

18. Composition selon la revendication 17 **caractérisée en ce que** ledit additif est présent à une teneur en poids variant de 1 à 30%.

19. Composition selon l'une des revendications 17 ou 18 **caractérisée en ce que** ledit diamide est présent en tant que matière active sèche à une teneur en poids variant de 0,2 à 8%, par rapport au poids de ladite composition de liant organique.

20. Composition selon l'une des revendications 17 à 19 **caractérisé en ce qu'**elle est une composition de revêtement, de mastic, de colle, d'adhésif, d'agent d'étanchéité, de moulage, ou de cosmétique.

21. Composition selon la revendication 20 **caractérisée en ce qu'**elle est réticulable, y compris auto-réticulable à température ambiante, ou **en ce qu'**elle est non réticulable.

22. Composition selon la revendication 21 **caractérisée en ce qu'**elle est réticulable mono-composante ou bi-composante.

23. Composition selon l'une des revendications 17 à 22 **caractérisée en ce que** ledit liant est sélectionné parmi au moins un système réactif époxy-amine, un polyester insaturé, un vinylester, une résine époxydée, une résine silicone réactive, une alkyde greffée par un polyester ou un polyamide ou modifiée diurée-diuréthane, ou une alkyde non greffée, un polyuréthane ou une silicone, un polyuréthane bi-composant réticulable, un polysiloxane, un polymère polysulfure, un polymère acrylique réactif, un oligomère multifonctionnel (méth)acrylate ou oligomère acrylique acrylé ou oligomère multifonctionnel allylique, un élastomère type SBR, polychloroprène ou caoutchouc butyle, ou un pré-polymère silané, de préférence un polyéther silané ou un polyuréthane silané, ou un polyéther-uréthane silané avec fonction -OH ou -CO₂H.

24. Composition selon l'une des revendications 20 à 23 **caractérisée en ce qu'**elle est une composition de mastic, de colle, d'adhésif ou d'agent d'étanchéité, qui est auto-réticulable, et à base de polyéther-silane ou de polyuréthane-silane.

25. Composition selon l'une des revendications 20 à 23 **caractérisée en ce qu'**elle est une composition de revêtement de protection et/ou de décoration et/ou de traitement de surface de divers substrats.

26. Composition selon la revendication 25 **caractérisée en ce que** ladite composition de revêtement est une composition de : peintures, vernis, gel coats, encres, ou plastisols à base de PVC.

27. Procédé de préparation d'une composition selon l'une des revendications 17 à 26 **caractérisé en ce qu'**il comprend les étapes suivantes:
i) addition dudit additif dans ladite composition de liant organique,
ii) homogénéisation du mélange avec un malaxeur et/ou mélangeur planétaire et/ou disperseur à haute vitesse, sans avoir aucun besoin, à cette étape, d'appliquer un traitement thermique d'activation.

28. Utilisation d'au moins un additif tel que défini selon l'une des revendications 1 à 14 ou obtenu par le procédé tel que défini selon l'une des revendications 15 ou 16, ou d'une composition telle que définie selon l'une des revendications 17 à 26, ou obtenue par le procédé tel que défini selon la revendication 27, dans des applications de revêtements, ou de mastics, de colles, d'adhésifs, d'agents d'étanchéité, de moulage, ou dans des applications cosmétiques.

29. Revêtements parmi les peintures, ou les vernis, ou les encres, ou les gels coats, ou les plastisols à base de PVC, ou des mastics, ou des colles, ou des adhésifs, ou des joints d'étanchéité, ou des composites, ou des pièces moulées, ou des stratifiés SMC/BMC, ou des produits cosmétiques **caractérisés en ce qu'**ils sont obtenus à partir d'au moins un additif tel que défini selon l'une des revendications 1 à 14 ou sont obtenus par l'utilisation telle que définie selon la revendication 28, ou sont obtenus à partir d'un additif obtenu par le procédé tel que défini selon l'une des revendications 15 ou 16, ou sont obtenus à partir d'une composition telle que définie selon l'une des revendications 17 à 26, ou à partir d'une composition obtenue par le procédé tel que défini selon la revendication 27.

## Claims

1. Rheology additive in the form of a pre-activated paste, **characterized in that** it comprises:
A) at least one fatty acid diamide, introduced in powder form, it being possible for said powder to optionally contain, in addition to said diamide, hydrogenated castor oil,
B) at least one organic plasticizer, said plasticizer being liquid at ambient temperature,
and **in that** the content by weight of said fatty acid diamide ranges from 10 to 40% and **in that** the maximum penetration value of said paste is less than 10 mm and preferably less than 5mm, measured according to standard ASTM D217.

2. Additive according to Claim 1, **characterized in that** said plasticizer is a polar organic plasticizer, comprising at least one ether group and/or one ester group and/or one epoxy group.

3. Additive according to Claim 2, **characterized in that** said plasticizer comprises at least one ether group, and **in that** it is chosen from polyethers which are homopolymers and/or copolymers of ethylene oxide and/or of propylene oxide and/or a blend of said polyethers and/or derivatives thereof, these derivatives comprising, *inter alia,* said polyethers blocked at the chain end with a C₁ to C₄ alkoxy group, or with a C₂ to C₄ ester group, said polyethers having a weight-average molecular weight Mw ranging from 150 to 6000.

4. Additive according to Claim 2, **characterized in that** said plasticizer comprises at least one ester group, and **in that** it is chosen from monoesters and/or polyesters obtained from C₄ to C₂₁ alcohols, which alcohols are optionally alkoxylated, and from mono- or polyacids with a functionality ranging from 1 to 4, selected from:
- organic acids chosen from aromatic acids having a chain length ranging from C₆ to C₁₀ and/or aliphatic acids having a chain length ranging from C₄ to C₁₈, or
- inorganic acids.

5. Additive according to Claim 4, **characterized in that** said mono- and/or polyesters are esters:
- of aromatic acids, chosen from phthalic and trimellitic esters,
- of aliphatic acids, chosen from adipic, citric, sebacic and azelaic esters, and
- of inorganic acids, chosen from sulphonic, sulphuric, sulphinic, phosphoric, phosphonic and phosphinic esters.

6. Additive according to Claim 2, **characterized in that** said plasticizer comprises at least one epoxy group chosen from epoxidized oils based on fatty acid.

7. Additive according to one of Claims 1 to 6, **characterized in that** said plasticizer is present in the absence of any other compound selected from alcohols with a molecular weight Mw < 150, or from polar aprotic solvents chosen from: N-methylpyrrolidone, N-ethylpyrrolidone, N-butylpyrrolidone, acetonitrile, N,N-dimethylformamide, N,N'-dimethylacetamide, dimethyl sulphoxide, N,N,N',N'-tetramethylurea, hexamethylphosphoramide, hexamethyl-phosphorous triamide or propylene carbonate.

8. Additive according to Claim 3, **characterized in that** said plasticizer is selected from polyethers which are homopolymers of propylene oxide with a weight-average molecular weight Mw ranging from 1000 to 3000, and/or derivatives thereof.

9. Additive according to Claim 5, **characterized in that** said plasticizer comprises at least one C₆ to C₁₀ aromatic acid ester group selected from mono- and/or dialkyl phthalates.

10. Additive according to Claim 9, **characterized in that** said plasticizer is at least one dialkyl phthalate, with said alkyls being identical or different and chosen from C₇ to C₁₈ alkyls.

11. Additive according to Claim 10, **characterized in that** said plasticizer is a diisoundecyl phthalate.

12. Additive according to Claim 5, **characterized in that** said plasticizer is a sulphonic ester selected from C₁₀ to C₂₁ alkyl sulphonates.

13. Additive according to one of Claims 1 to 12, **characterized in that** said fatty acid diamide is obtained by polycondensation between at least one C₂ to C₁₂ primary diamine and at least one monocarboxylic acid with a chain length of C₃ to C₂₂, it being possible for the reaction product to be optionally diluted in hydrogenated castor oil, and in this case to a content ranging from 10 to 100% by weight, relative to the diamide + hydrogenated castor oil total.

14. Additive according to one of Claims 1 to 13, **characterized in that** it can obtained by means of a process comprising the following steps:
i) gradual dispersion of said diamide in powder form, in said plasticizer until a homogeneous dispersion is obtained, at ambient temperature controlled by virtue of regulation of the temperature,
ii) maintenance of the homogeneous dispersion obtained during step i) under at least one set of stationary heating conditions with a corresponding temperature ranging from 50 to 120°C, for a period of time of from 1 to 100 hours.

15. Process for preparing the additive as defined according to one of Claims 1 to 14, **characterized in that** it comprises the following steps:
i) gradual dispersion of said diamide in powder form, in the plasticizer until a homogeneous dispersion is obtained, at ambient temperature controlled by virtue of regulation of the temperature, the diamide and the plasticizer being as defined according to one of Claims 1 to 14,
ii) maintenance of the homogeneous dispersion obtained during step i) under at least one set of stationary heating conditions with a corresponding temperature ranging from 50 to 120°C, for a period of time of from 1 to 100 hours.

16. Process according to Claim 15, **characterized in that**, at the end of step ii), the maximum penetration value of the paste formed is less than 10 mm, measured according to standard ASTM D 217.

17. Organic binder composition, **characterized in that** it comprises, in addition to said binder, at least one additive as defined according to one of Claims 1 to 14, or obtained by means of a process as defined according to either of Claims 15 and 16.

18. Composition according to Claim 17, **characterized in that** said additive is present at a content by weight ranging from 1 to 30%.

19. Composition according to either of Claims 17 and 18, **characterized in that** said diamide is present as dry active matter at a content by weight ranging from 0.2 to 8%, relative to the weight of said organic binder composition.

20. Composition according to one of Claims 17 to 19, **characterized in that** it is a coating, mastic, glue, adhesive, leaktightness agent, moulding or cosmetic composition.

21. Composition according to Claim 20, **characterized in that** it is crosslinkable, including self-crosslinkable at ambient temperature, or **in that** it is noncrosslinkable.

22. Composition according to Claim 21, **characterized in that** it is single-component or two-component crosslinkable.

23. Composition according to one of Claims 17 to 22, **characterized in that** said binder is selected from at least one epoxy/amine reactive system, an unsaturated polyester, a vinyl ester, an epoxidized resin, a reactive silicone resin, an alkyd grafted with a polyester or a polyamide or an alkyd modified with diurea/diurethane, or an ungrafted alkyd, a polyurethane or a silicone, a crosslinkable two-component polyurethane, a polysiloxane, a polysulphide polymer, a reactive acrylic polymer, a (meth)acrylate polyfunctional oligomer or acrylated arylic oligomer or allylic polyfunctional oligomer, a butyl rubber, polychloroprene or SBR type elastomer, or a silylated prepolymer, preferably a silylated polyether or a silylated polyurethane, or a silylated polyether/urethane with an -OH or -CO₂H function.

24. Composition according to one of Claims 20 to 23, **characterized in that** it is a mastic, glue, adhesive or leaktightness agent composition which is self-crosslinkable and based on polyether/silane or on polyurethane/silane.

25. Composition according to one of Claims 20 to 23, **characterized in that** it is a composition for the protective and/or decorative and/or surface-treatment coating of various substrates.

26. Composition according to Claim 25, **characterized in that** said coating composition is a composition of:
paints, varnishes, gel coats, inks or plastisols based on PVC.

27. Process for preparing a composition according to one of Claims 17 to 26, **characterized in that** it comprises the following steps:
i) addition of said additive to said organic binder composition,
ii) homogenization of the mixture with a kneader and/or planetary mixer and/or high-speed disperser, without having any need, in this step, to apply a heat activation treatment.

28. Use of at least one additive as defined according to one of Claims 1 to 14 or obtained by means of the process as defined according to either of Claims 15 and 16, or of a composition as defined according to one of Claims 17 to 26, or obtained by means of the process as defined according to Claim 27, in coating applications, or mastic, glue, adhesive, leaktightness agent or moulding applications, or in cosmetic applications.

29. Coatings from paints, or varnishes, or inks, or gel coats, or plastisols based on PVC, or mastics, or glues, or adhesives, or leaktight seals, or composites, or moulded components, or SMC/BMC laminates, or cosmetic products, **characterized in that** they are obtained from at least one additive as defined according to one of Claims 1 to 14 or are obtained through the use as defined according to Claim 28, or are obtained from an additive obtained by means of the process as defined according to either of Claims 15 or 16, or are obtained from a composition as defined according to one of Claims 17 to 26, or from a composition obtained by means of the process as defined according to Claim 27.

## Patentansprüche

1. Rheologieadditiv in Form einer voraktivierten Paste, **dadurch gekennzeichnet, dass** es Folgendes umfasst:
A) mindestens ein Fettsäurediamid, das in Form von Pulver eingeführt wird, wobei das Pulver, neben dem Diamid, optional hydrogeniertes Rizinusöl enthalten kann,
B) mindestens einen organischen Weichmacher, wobei der Weichmacher bei Umgebungstemperatur flüssig ist,
und **dadurch**, dass das Gewichtsverhältnis des Fettsäurediamids von 10 bis 40 % variiert und **dadurch**, dass der Wert der maximalen Penetration der Paste, gemessen gemäß ASTM-Norm D217, kleiner als 10 mm und bevorzugt kleiner als 5 mm ist.

2. Additiv nach Anspruch 1, **dadurch gekennzeichnet, dass** der Weichmacher ein polarer organischer Weichmacher ist, der mindestens eine Ethergruppe und/oder eine Estergruppe und/oder eine Epoxygruppe umfasst.

3. Additiv nach Anspruch 2, **dadurch gekennzeichnet, dass** der Weichmacher mindestens eine Ethergruppe umfasst und **dadurch**, dass er ausgewählt ist aus den Polyethern, die Ethylenoxid- und/oder Propylenoxid-Homopolymere und/oder -Copolymere sind und/oder einem Gemisch aus diesen Polyethern und/oder deren Derivaten, wobei diese Derivate unter anderem die Polyether umfassen, die am Kettenende durch eine C₁- bis C₄-Alkoxygruppe oder durch eine C₂- bis C₄-Estergruppe blockiert sind, wobei die Polyether ein gewichtsgemitteltes Molekulargewicht Mw von 150 bis 6.000 aufweisen.

4. Additiv nach Anspruch 2, **dadurch gekennzeichnet, dass** der Weichmacher mindestens eine Estergruppe umfasst, ausgewählt aus den Monoestern und/oder Polyestern, erhalten aus C₄- bis C₂₁-Alkoholen, gegebenenfalls alkoxylierten Alkoholen, und aus Mono- oder Polysäuren mit einer Funktionalität, die von 1 bis 4 reicht, gewählt aus:
- organischen Säuren, ausgewählt aus den aromatischen Säuren mit einer Kettenlänge im Bereich von C₆ bis C₁₀ und/oder den aliphatischen Säuren mit einer Kettenlänge im Bereich von C₄ bis C₁₈, oder
- den mineralischen Säuren.

5. Additiv nach Anspruch 4, **dadurch gekennzeichnet, dass** die Mono- und/oder Polyester:
- Ester aromatischer Säuren, ausgewählt aus den Phthalsäure- und Trimellithsäureestern,
- Ester aliphatischer Säuren, ausgewählt aus den Adipinsäure- Citronensäure-, Sebacinsäure-, Azelainsäureestern, und
- Ester mineralischer Säuren sind, ausgewählt aus den Sulfonsäure-, Schwefelsäure-, Sulfinsäure-, Phosphorsäure-, Phosphonsäure- und Phosphinsäureestern.

6. Additiv nach Anspruch 2, **dadurch gekennzeichnet, dass** der Weichmacher mindestens eine Epoxygruppe umfasst, ausgewählt aus den epoxidierten Ölen auf Fettsäurebasis.

7. Additiv nach einem der Ansprüche 1 bis 6, **dadurch gekennzeichnet, dass** der Weichmacher in Abwesenheit jeder anderen Verbindung vorhanden ist, gewählt aus den Alkoholen mit einem Molekulargewicht Mw <150 oder aus den polaren aprotischen Lösemitteln, ausgewählt aus: N-Methylpyrrolidon, N-Ethylpyrrolidon, N-Butylpyrrolidon, Acetonitril, N,N-Dimethylformamid, N,N'-Dimethylacetamid, Dimethylsulfoxid, N,N,N',N'-Tetramethylharnstoff, Hexamethylphosphoramid, Hexamethylphosphortriamid oder Propylencarbonat.

8. Additiv nach Anspruch 3, **dadurch gekennzeichnet, dass** der Weichmacher gewählt ist aus den Polyethern, die Propylenoxid-Homopolymere mit einem gewichtsgemittelten Molekulargewicht Mw im Bereich von 1.000 bis 3.000 sind, und/oder deren Derivaten.

9. Additiv nach Anspruch 5, **dadurch gekennzeichnet, dass** der Weichmacher mindestens eine aromatische C₆- bis C₁₀-Säureestergruppe umfasst, gewählt aus den Mono- und/oder den Dialkylphthalaten.

10. Additiv nach Anspruch 9, **dadurch gekennzeichnet, dass** der Weichmacher mindestens ein Dialkylphthalat ist, wobei die Alkyle gleich oder verschieden und aus den C₇- bis C₁₈-Alkylen ausgewählt sind.

11. Additiv nach Anspruch 10, **dadurch gekennzeichnet, dass** der Weichmacher ein Diisoundecylphthalat ist.

12. Additiv nach Anspruch 5, **dadurch gekennzeichnet, dass** der Weichmacher ein Sulfonester ist, gewählt aus den C₁₀- bis C₂₁-Alkoylsulfonaten.

13. Additiv nach einem der Ansprüche 1 bis 12, **dadurch gekennzeichnet, dass** das Fettsäurediamid durch Polykondensation mindestens eines primären C₂- bis C₁₂-Diamins und mindestens einer Monocarbonsäure mit einer Kettenlänge von C₃- bis C₂₂ erhalten wird, wobei das Reaktionsprodukt optional in hydriertem Rizinusöl verdünnt sein kann, und in diesem Fall mit einem Anteil, der, bezogen auf das Gesamtgewicht von Diamid + hydriertem Rizinusöl, von 10 bis 100 Gew.-% variiert.

14. Additiv nach einem der Ansprüche 1 bis 13, **dadurch gekennzeichnet, dass** es geeignet ist, durch ein Verfahren erhalten zu werden, das die folgenden Schritte umfasst:
i) allmähliche Dispersion des Diamids in Form von Pulver in dem Weichmacher bis zum Erhalt einer homogenen Dispersion bei Umgebungstemperatur, die mithilfe einer Temperatursteuerung kontrolliert wird,
ii) Aufrechterhalten der homogenen Dispersion, die während des Schritts i) erhalten wurde, in mindestens einer Isotherme mit einer entsprechenden Temperatur im Bereich von 50 bis 120 °C für eine Dauer von 1 bis 100 Stunden.

15. Verfahren zur Herstellung des Additivs wie in einem der Ansprüche 1 bis 14 definiert, **dadurch gekennzeichnet, dass** es die folgenden Schritte umfasst:
i) allmähliche Dispersion des Diamids in Form von Pulver in dem Weichmacher bis zum Erhalt einer homogenen Dispersion bei Umgebungstemperatur, die mithilfe einer Temperatursteuerung kontrolliert wird, wobei das Diamid und der Weichmacher so sind, wie in einem der Ansprüche 1 bis 14 definiert.
ii) Aufrechterhalten der homogenen Dispersion, die während des Schritts i) erhalten wurde, in mindestens einer Isotherme mit einer entsprechenden Temperatur im Bereich von 50 bis 120 °C für eine Dauer von 1 bis 100 Stunden.

16. Verfahren nach Anspruch 15, **dadurch gekennzeichnet, dass** am Ende des Schritts ii) der Wert der maximalen Penetration der gebildeten Paste, gemessen gemäß ASTM-Norm D 217, kleiner als 10 mm ist.

17. Organische Bindemittelzusammensetzung, **dadurch gekennzeichnet, dass** sie neben dem Bindemittel mindestens ein Additiv umfasst, wie in einem der Ansprüche 1 bis 14 definiert, oder durch ein Verfahren erhalten, wie in einem der Ansprüche 15 oder 16 definiert.

18. Zusammensetzung nach Anspruch 17, **dadurch gekennzeichnet, dass** das Additiv in einem Anteil vorhanden ist, der von 1 bis 30 Gew.-% variiert.

19. Zusammensetzung nach einem der Ansprüche 17 oder 18, **dadurch gekennzeichnet, dass** das Diamid als aktive Trockensubstanz in einem Gewichtsanteil vorhanden ist, der, bezogen auf das Gewicht der organischen Bindemittelzusammensetzung, von 0,2 bis 8 Gew.-% variiert.

20. Zusammensetzung nach einem der Ansprüche 17 bis 19, **dadurch gekennzeichnet, dass** sie eine Beschichtungs-, Kitt-, Leim-, Klebstoff-, Dichtungsmittel-, Formmassen- oder Kosmetikzusammensetzung ist.

21. Zusammensetzung nach Anspruch 20, **dadurch gekennzeichnet, dass** sie bei Umgebungstemperatur vernetzbar, einschließlich selbstvernetzbar, ist oder **dadurch**, dass sie nicht vernetzbar ist.

22. Zusammensetzung nach Anspruch 21, **dadurch gekennzeichnet, dass** sie einkomponentig oder zweikomponentig vernetzbar ist.

23. Zusammensetzung nach einem der Ansprüche 17 bis 22, **dadurch gekennzeichnet, dass** das Bindemittel ausgewählt ist aus mindestens einem reaktiven Epoxy-Aminsystem, einem ungesättigten Polyester, einem Vinylester, einem expoxidierten Harz, einem reaktiven Silikonharz, einem mit einem Polyester oder einem Polyamid gepfropften Alkyd oder einem mit Diharnstoff/Diurethan modifizierten Alkyd oder einem nicht gepfropften Alkyd, einem Polyurethan oder einem Silicon, einem zweikomponentig vernetzbaren Polyurethan, einem Polysiloxan, einem Polysulfidpolymer, einem reaktiven Acrylpolymer, einem multifunktionellen (Meth)acrylatoligomer oder einem acrylierten Acryloligomer oder einem multifunktionellen Allyloligomer, einem Elastomer des Typs SBR, Polychloropren oder Butylkautschuk, oder einem silanierten Präpolymer, bevorzugt einem silanierten Polyether oder einem silanierten Polyurethan, oder einem silanierten Polyether-Urethan mit -OH- oder -CO₂H-Funktion.

24. Zusammensetzung nach einem der Ansprüche 20 bis 23, **dadurch gekennzeichnet, dass** sie eine Kitt-, Leim-, Klebstoff- oder Dichtungsmittelzusammensetzung ist, die selbstvernetzbar und auf der Basis von Polyethersilan oder Polyurethansilan ist.

25. Zusammensetzung nach einem der Ansprüche 20 bis 23, **dadurch gekennzeichnet, dass** sie eine Beschichtungszusammensetzung zum Schutz und/oder zur Dekoration und/oder zur Oberflächenbehandlung verschiedener Substrate ist.

26. Zusammensetzung nach Anspruch 25, **dadurch gekennzeichnet, dass** die Beschichtungszusammensetzung eine Zusammensetzung aus Farben, Lacken, Gelcoats, Tinten oder Plastisolen auf PVC-Basis ist.

27. Verfahren zur Herstellung einer Zusammensetzung wie in einem der Ansprüche 17 bis 26 definiert, **dadurch gekennzeichnet, dass** es die folgenden Schritte umfasst:
i) Zugeben des Additivs in die organische Bindemittelzusammensetzung,
ii) Homogenisieren des Gemischs mit einem Rührer und/oder einem Planetenmischer und/oder einem Hochgeschwindigkeitsdispergierer, ohne dass in diesem Schritt die Anwendung irgendeiner thermischen Aktivierungsbehandlung erforderlich wäre.

28. Verwendung mindestens eines Additivs, wie in einem der Ansprüche 1 bis 14 definiert oder durch das Verfahren erhalten, wie in einem der Ansprüche 15 oder 16 definiert, oder einer Zusammensetzung, wie in einem der Ansprüche 17 bis 26 definiert, oder durch das Verfahren erhalten, wie in Anspruch 27 definiert, in Beschichtungs- oder Kitt-, Leim-, Klebstoff-, Dichtungsmittel-, Formmassenanwendungen oder in kosmetischen Anwendungen.

29. Beschichtungen aus Farben oder Lacken oder Tinten oder Gelcoats oder Plastisolen auf PVC-Basis oder Kitten oder Leimen oder Klebstoffen oder Dichtungen oder Verbundstoffen oder Formteilen oder SMC/BMC-Laminaten oder kosmetischen Produkten, **dadurch gekennzeichnet, dass** sie ausgehend von mindestens einem Additiv erhalten werden, wie in einem der Ansprüche 1 bis 14 definiert, oder durch eine Verwendung erhalten werden, wie in Anspruch 28 definiert, oder ausgehend von einem Additiv erhalten werden, das durch das Verfahren erhalten wird, wie in einem der Ansprüche 15 oder 16 definiert, oder ausgehend von einer Zusammensetzung, wie in einem der Ansprüche 17 bis 26 definiert, oder ausgehend von einer Zusammensetzung erhalten werden, die durch das Verfahren erhalten wird, wie in Anspruch 27 definiert.
